# EUROPEAN PATENT APPLICATION

(11) **EP 4 215 122 A2**
(43) Date of publication of application: **26.07.2023**
(21) Application number: 21869697.9
(22) Date of filing: 15.09.2021
(51) Int. Cl.: A61B 10/00, C12M 1/30, G01N 1/10, A61B 10/02, G01N 1/02

(54) **SAMPLE COLLECTION STICK**

(30) Priority: 16.09.2020 KR 20200119514; 05.02.2021 KR 20210017056; 05.02.2021 KR 20210017061; 14.09.2021 KR 20210122448
(71) Applicant: Bionlifescience, Inc., Namyangju-si, Gyeonggi-do 12106 (KR)
(72) Inventor: GOH, Chang Wook, Namyangju-si Gyeonggi-do 12095 (KR); JEONG, Joong Hwan, Bucheon-si Gyeonggi-do 14500 (KR); KIM, Bong Yoon, Goyang-si Gyeonggi-do 10469 (KR)
(74) Representative: dompatent von Kreisler Selting Werner - Partnerschaft von Patent- und Rechtsanwälten mbB
(86) International application number: PCT/KR2021/012567
(87) International publication number: WO 2022/060075

(57) **Abstract**

The present invention relates to a sample collection stick. According to the present invention, a sample collection part includes a screw blade supported by a hub and formed in a spiral shape around the lengthwise central axis of the hub, and multiple concaves ( 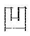 ) and convexes ( 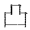 ) corresponding to a collection groove are alternately formed on the outer rim of the screw blade. Therefore, disclosed is a technique capable of increasing the amount of samples collected from an examinee and increasing the amount of collected samples dissolved or dispersed into a reagent or a solution from a sample collection stick.

## Description

### [Technical Field]

The present invention relates to a specimen collection stick, and more particularly to a specimen collection stick capable of scraping a specimen, such as saliva in the oral cavity or the nasal cavity of a subject, thereby collecting the specimen, such as saliva in the oral cavity or the nasal cavity of the subject.

### [Background Art]

A microbiological examination is an examination for determining whether a specimen has been contaminated by disease-causing germs. That is, when it is thought that a cause of illness is a microorganism, the microbiological examination is performed in order to establish a method of diagnosing, treating, and preventing the same. The microbiological examination is performed to determine whether a subject has been contaminated by disease-causing germs, such as a colon bacillus, a typhoid bacillus, staphylococcus, and pseudomonas.

In general, a specimen is collected from the body of the subject in order to perform the microbiological examination. The collected specimen is mixed with a reagent or solution for examination, and then examination is performed to determine whether there are disease-causing germs.

In order to collect a specimen, such as saliva or a bodily fluid, from bodily tissue of the subject, a tool called a collection stick, a brush, or a swab is used. That is, the swab for specimen collection is introduced into the body of the subject, a specimen is adhered to the swab, and the swab is withdrawn, whereby the specimen is collected.

In the conventional swab for specimen collection, a circular collection unit is formed at an end of the swab, which is formed in the shape of a bar. The collection unit of the swab is provided with a fiber layer, which is formed by attaching small-sized microfibers to the collection unit. A specimen permeates the fiber layer formed on the collection unit of the conventional swab, and the collection unit of the swab is withdrawn from the subject, whereby the specimen is collected.

In the conventional swab for specimen collection used to collect the specimen, however, it is necessary for the swab to remain inserted in the oral cavity or the nasal cavity of the subject for a predetermined time or more such that saliva or the like permeates the fiber layer, whereby the subject may experience great discomfort. Above all, a part of the fiber layer included in the swab for specimen collection may be separated from the collection unit of the swab and may remain in the body of the subject. When foreign matter, such as the fiber layer, remains in the body of the subject, a medically serious problem may occur. In addition, the amount of the specimen that is collected using the conventional swab is not sufficient, whereby examination accuracy may be lowered.

Therefore, there is a need for technology capable of solving such problems.

### [Disclosure]

### [Technical Problem]

The present invention has been made in view of the above problems, and it is an object of the present invention to provide a specimen collection stick capable of increasing the amount of a specimen that is collected during specimen collection, shortening time necessary for specimen collection, and preventing a problem in which fibers constituting a fiber layer of the conventional swab are separated from the swab.

### [Technical Solution]

A specimen collection stick according to an embodiment of the present invention to accomplish the above object includes a rod-shaped or bar-shaped support unit having a predetermined length and a specimen collection unit located at a front end of the support unit, the specimen collection unit being configured to collect a specimen in a body of a subject that comes into contact with the specimen collection unit, wherein the specimen collection unit includes a hub coupled to the front of the support unit and a screw blade disposed on an outer surface of the hub, the screw blade being supported by the hub, the screw blade being spirally formed along a central axis of the hub in a longitudinal direction thereof, a plurality of concave parts corresponding to collection recesses and a plurality of convex parts are alternately formed at an outer edge of the screw blade, and the screw blade or the hub is made of a flexible or elastic material.

A coupling projection or coupling protrusion may be formed at an outer circumferential surface of the support unit so as to protrude therefrom, or a lid fastening groove may be formed in the outer circumferential surface of the support unit, such that the support unit can be fixed to a lid of a specimen container configured to receive the specimen collection unit therein by coupling.

A specimen collection stick according to another embodiment of the present invention to accomplish the above object includes a rod-shaped or bar-shaped support unit having a predetermined length and a specimen collection unit located at a front end of the support unit, the specimen collection unit being configured to collect a specimen in a body of a subject that comes into contact with the specimen collection unit, wherein the specimen collection unit includes a hub coupled to the front of the support unit and a plurality of collection disks disposed on an outer surface of the hub, the collection disks being supported by the hub, the collection disks being arranged in a longitudinal direction of the hub, a plurality of concave parts corresponding to collection recesses and a plurality of convex parts are alternately formed at an outer edge of each of the plurality of collection disks, and each of the collection disks or the hub is made of a flexible or elastic material.

A coupling projection or coupling protrusion may be formed at an outer circumferential surface of the support unit so as to protrude therefrom, or a lid fastening groove may be formed in the outer circumferential surface of the support unit, such that the support unit can be fixed to a lid of a specimen container configured to receive the specimen collection unit therein by coupling.

### [Advantageous Effects]

A specimen collection stick according to the present invention has effects in that a flocking process, which is required to manufacture the conventional swab for specimen collection, is not necessary, whereby it is possible to reduce the manufacturing cost of the specimen collection stick, and in that there is no fiber layer in the specimen collection stick, whereby it is possible to prevent a problem in which the fiber layer is separated from the specimen collection stick and remains in the body of a subject, unlike the conventional swab for specimen collection, and therefore it is possible to improve examination safety.

Also, in the specimen collection stick according to the present invention, it is possible to increase the amount of the specimen that is collected from the body of the subject, and it is possible to reduce time necessary for specimen collection, since absorption time for which the specimen permeates the fiber layer is not necessary, unlike the conventional swab. Consequently, the specimen collection stick according to the present invention has effects in that it is possible to shorten time necessary for specimen collection, to reduce inconvenience of the subject during specimen collection, and to improve examination accuracy with an increase in amount of the specimen that is collected.

### [Description of Drawings]

FIG. 1 is a perspective view schematically showing a specimen collection stick according to an embodiment of the present invention.
FIG. 2 is a perspective view schematically showing a specimen collection unit of the specimen collection stick according to the embodiment of the present invention.
FIG. 3 is a front view schematically showing the specimen collection unit of the specimen collection stick according to the embodiment of the present invention.
FIG. 4 is a partial sectional view schematically showing a hub and a screw blade in the specimen collection stick according to the embodiment of the present invention.
FIG. 5 is a perspective view schematically showing a specimen collection stick according to an applied embodiment of the present invention.
FIG. 6 is a perspective view schematically showing a specimen collection unit of the specimen collection stick according to the applied embodiment of the present invention.
FIG. 7 is a front view schematically showing the specimen collection unit of the specimen collection stick according to the applied embodiment of the present invention.
FIG. 8 is a partial sectional view schematically showing a hub and a collection blade in the specimen collection stick according to the applied embodiment of the present invention.

### [Best Mode]

The present invention may be variously changed and may have several embodiments, and therefore specific embodiments will be described in detail while being illustrated in the drawings. However, the present invention is not limited to the specific embodiments, and it should be understood that the present invention includes all alterations, equivalents, and substitutions falling within the idea and technical scope of the present invention. The present embodiments are provided to more specifically describe the present invention to a person having ordinary skill in the art to which the present invention pertains. Consequently, the shape of each element in the drawings may be exaggerated for clearer description. In describing the present invention, a detailed description of related known technology will be omitted when the same may obscure the subject matter of the present invention.

Although the terms, such as "first" and "second," may be used to describe various elements, the elements must not be defined by the terms. The terms are used only for the purpose of distinguishing one element from another and describing the same so as to be understood.

The terms used in the present invention are used only to describe a specific embodiment, not to define the present invention. Singular forms include plural forms unless mentioned otherwise.

It should be understood that the terms "comprises," "has," etc. specify the presence of stated features, numbers, steps, operations, elements, components, or combinations thereof described in this specification, but do not preclude the presence or addition of one or more other features, numbers, steps, operations, elements, components, or combinations thereof.

Hereinafter, embodiments of the present invention will be described with reference to the accompanying drawings, and the present invention will be described in detail to the extent to which a person having ordinary skill in the art to which the present invention pertains can easily implement the present invention. However, the present invention may be implemented in various different forms and is not limited to the embodiments described herein. Similar parts may be denoted by the same reference numerals throughout the specification.

Hereinafter, embodiments of a specimen collection stick according to the present invention will be described with reference to the drawings.

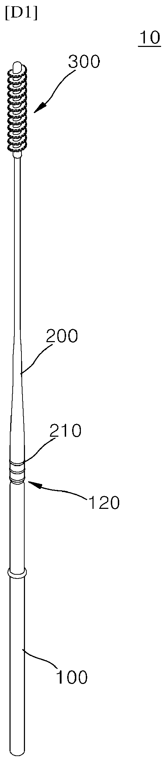

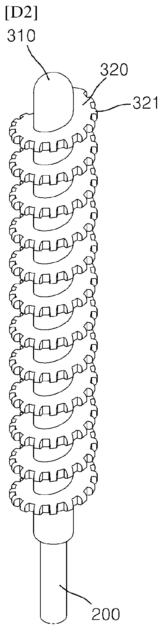

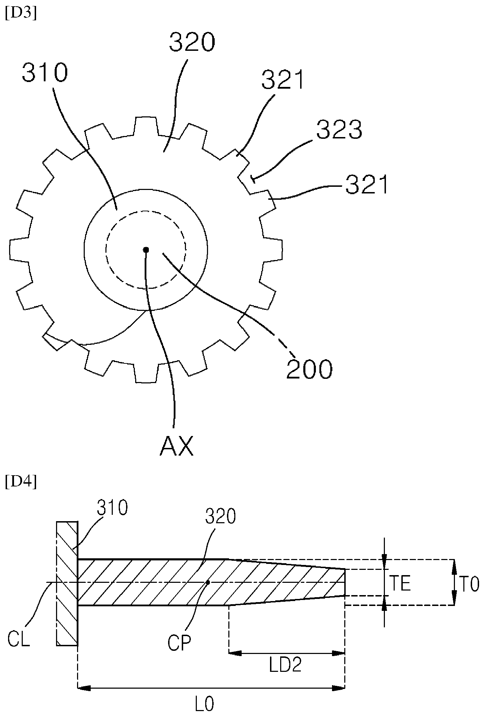

FIG. 1 is a perspective view schematically showing a specimen collection stick according to an embodiment of the present invention, FIG. 2 is a perspective view schematically showing a specimen collection unit of the specimen collection stick according to the embodiment of the present invention, and FIG. 3 is a front view schematically showing the specimen collection unit of the specimen collection stick according to the embodiment of the present invention.

Referring to FIGs. 1 to 3, the specimen collection stick according to the embodiment of the present invention includes a support unit 200 and a specimen collection unit 300, and may further include a handle unit.

First, the support unit 200 is formed so as to have a shape, such as a rod or a bar, and has a predetermined length, as shown in the figures. The specimen collection unit 300 is coupled to a front end of the support unit 200. The support unit 200 supports the specimen collection unit 300. The handle unit is connected to the rear of the support unit 200. The support unit 200 supports the specimen collection unit 300 such that the specimen collection unit can be introduced into the body of a subject.

It is sufficient for the support unit 200 to support the specimen collection unit 300 such that the specimen collection unit can be introduced into the body of the subject in order to perform specimen collection, and the shape of the support unit is not limited to a specific shape.

Furthermore, it is preferable for the support unit 200 to be made of a polymer material that exhibits flexibility and elasticity such that the support unit can return to the original shape thereof while being flexibly deformed by force applied thereto.

Meanwhile, when the specimen collection unit 300 and the support unit 200 of the specimen collection stick 10 are withdrawn from a specimen container (not shown) using a tool, such as a pincette, it is necessary to wash the pincette in advance. In addition, after the support unit 200 of the specimen collection stick 10 is withdrawn from the specimen container using the pincette and the specimen collection unit 300 and the support unit 200 of the specimen collection stick 10 are located at a position intended by an examiner, it is necessary to wash the used pincette again.

Since it is necessary to wash the pincette whenever the pincette is used in the process of withdrawing the specimen collection stick from the specimen container, as described above, specimen examination efficiency is lowered. In order to withdraw the specimen collection unit 300 and the support unit 200 of the specimen collection stick 10 from the specimen container without using the pincette, therefore, the following construction may be provided.

That is, a coupling projection or coupling protrusion 210 may be formed at an outer surface of the support unit 200 so as to protrude from the outer surface thereof such that the coupling projection or coupling protrusion is fixed to a cover or lid of the specimen container by coupling.

The cover or lid (not shown) of the specimen container (not shown) may be provided at an inside thereof with a fixing groove or fitting projection (not shown) configured to allow the support unit 200 of the specimen collection stick to be fixed to the cover or lid (not shown) of the specimen container therethrough. The coupling projection or coupling protrusion 210 of the support unit may be coupled to the fixing groove or fitting projection provided at the inside of the cover or lid of the specimen container by engagement or fitting.

Since the support unit 200 can be fixed to the cover or lid of the specimen container, as described above, it is possible to easily withdraw the support unit 200 from the specimen container together with the cover or lid of the specimen container when the cover or lid is removed from the specimen container without using a tool, such as a pincette, which is preferable.

FIG. 1 shows a ring-shaped coupling projection or coupling protrusion 210 formed on the outer surface of the support unit 200 in a circumferential direction thereof as an illustrative example of the coupling projection or coupling protrusion 210. As shown in FIG. 1, it is also preferable for one or more ring-shaped coupling projections or coupling protrusions 210 to be provided on the outer surface of the support unit 200 at the rear of the support unit 200.

As long as the support unit 200 can be coupled to the cover of the specimen container, as described above, it is possible to improve convenience in use, which is preferable.

As previously described, the handle unit 100 is located at the rear of the support unit 200. That is, a rear end of the support unit 200 is connected to the handle unit 100.

The handle unit 100 is connected to the rear end of the support unit 200, and the handle unit has a predetermined length such that the handle unit can be gripped by the hand or fingers of the examiner.

As shown in the figures, the handle unit 100 is formed in the shape of a rod or a bar having a predetermined length. The handle unit 100 is gripped by the hand or fingers of the examiner. The examiner may perform control such that the specimen collection unit 300 is introduced or inserted into the body of the subject using the handle unit 100.

Consequently, the specimen collection unit 300 may be introduced or inserted into the body of the subject through the oral cavity or the nasal cavity of the subject under control of the examiner using the handle unit 100.

As shown in the figures, it is preferable for a segment groove 120, such as a segment joint, capable of distinguishing between the handle unit 100 and the support unit 200 to be provided between the handle unit 100 and the rear end of the support unit 200. That is, it is preferable for the segment groove 120 to be provided between the handle unit 100 and the support unit 200 such that the handle unit 100 and the support unit 200 can be separated from each other.

After the specimen collection unit 300 is withdrawn from the body of the subject according to the intention of the examiner, who is a user, the specimen collection unit 300 and the support unit 200 are received in the specimen container.

Since it is not necessary to receive the handle unit 100 in the specimen container at this time, it is preferable to separate the handle unit 100 from the support unit 200. As shown in the figures, therefore, it is preferable for the segment joint or segment groove 120 to be provided between the support unit 200 and the handle unit 100 such that the support unit 200 and the handle unit 100 can be separated from each other as needed.

After the support unit 200 and the handle unit 100 are separated from each other, the support unit 200 may be coupled to the cover or lid of the specimen container, as previously described. It is preferable for the coupling projection or coupling protrusion 210 to be formed at an outer circumferential surface of the support unit so as to protrude therefrom or for a lid fastening groove to be formed in the outer circumferential surface of the support unit such that the support unit can be fixed to the cover or lid of the specimen container, in which the specimen collection unit is received, by coupling.

The specimen collection unit 300 is a part that is inserted or introduced into the body of the subject so as to come into contact with a specimen. That is, the specimen collection unit is a part that collects the specimen. The specimen collection unit 300 is located at the front end of the support unit 200 in order to gather or collect a specimen in the body of the subject that comes into contact with the specimen collection unit.

The specimen collection unit 300 includes a hub 310 and a screw blade 320.

The hub 310 is coupled to the front of the support unit 200. It is preferable for the hub 310 and the support unit 200 to be coupled to each other such that a central axis of the hub in a longitudinal direction thereof and a central axis of the support unit 200 in a longitudinal direction thereof are aligned with each other. The hub 310 is coupled to or formed at the support unit 200 such that a front portion of the support unit 200 is inserted into the hub 310. In addition, the hub 310 supports the screw blade 320 disposed at the outside thereof.

The screw blade 320 is disposed at the outside of the hub 310, is supported by the hub 310, and is spirally formed along the central axis of the hub in the longitudinal direction thereof.

The spirally formed screw blade 320 is disposed at the outside of the hub 310 such that a specimen brought into contact with the screw blade is withdrawn from the body of the subject in a state of being adhered to or held by the screw blade.

It is preferable for the hub 310 to be made of a flexible or elastic material. That is, the hub may be made of a polymer material that exhibits flexibility and elasticity or an elastomer material.

As shown in the figures, the hub 310 may be formed in the shape of a bar having a circular section; however, the present invention is not limited thereto.

The specimen collection unit 300 includes a screw blade 320 spirally formed along the central axis of the support unit 200 or the hub 310 in the longitudinal direction thereof.

That is, the specimen collection unit 300 includes a spiral screw blade 320 configured to allow a specimen brought into contact therewith to be adhered thereto or to be held thereby and to be withdrawn from the body of the subject in that state.

The screw blade 320 may be formed around the hub 310 on an outer circumferential surface of the hub 310. The screw blade 320 thus formed is supported by the hub 310.

The hub 310 configured to support the screw blade 320 is coupled to the front portion of the support unit 200. Consequently, the hub 310 supports the screw blade 320 while being supported by the support unit 200. When the screw blade 320 is included in the specimen collection unit 300, as described above, the surface area of the specimen collection unit 300 that contacts the specimen is increased. Consequently, the amount of the specimen that is collected is increased.

An outer edge of the spiral screw blade 320 may be formed at a predetermined pitch. On the assumption that an arbitrary pitch of the screw blade 320 is a first pitch and the next pitch of the screw blade after being rotated 360 degrees about the central axis of the hub 310 in the longitudinal direction thereof from the first pitch is a second pitch, a gap, through which the outer circumferential surface of the hub 310 is exposed outwards, is provided between the first pitch and the second pitch. Even in the gap, the specimen collection unit may come into contact with a specimen in the body of the subject, and the specimen may be withdrawn from the body of the subject in a state of being held in the gap.

In short, a specimen may be adhered and held between the screw blade and the outer circumferential surface of the hub, and the specimen may be withdrawn from the body of the subject in that state.

Each of the screw blades 320 is flexible and elastic. Consequently, each of the plurality of screw blades 320 abuts bodily tissue in the body of the subject. The examiner swings the specimen collection unit 300 in the body of the subject using the handle unit 100 such that the screw blade 320 can scrape or sweep the bodily tissue in which the specimen is present while abutting the bodily tissue. As a result, the specimen is adhered to the surface of the screw blade 320 or is held in the gap between neighboring pitches of the screw blade 320.

That is, the specimen is adhered to the surface of the screw blade 320 or is held in the gap between the neighboring pitches of the screw blade 320 in the state in which the specimen collection unit 300 is inserted in the body of the subject, whereby the specimen remains on the specimen collection unit 300 when the specimen collection unit 300 is withdrawn from the body of the subject.

The gap between the neighboring pitches of the screw blade 320 may also be referred to as a collection space or a collection gap.

In the specimen collection unit 300, therefore, the specimen brought into contact with the screw blade 320 or the hub 310 may remain held by the screw blade 320 or adhered thereto.

A plurality of concave parts 323 corresponding to the collection recesses and a plurality of convex parts 321 may be alternately formed at the outer edge of the spiral screw blade 320. In other words, a plurality of collection recesses 323 may be formed at the outer edge of the spiral screw blade 320, which is preferable. Here, the concave and convex parts 321 and 323 or the collection recesses 323 may have various shapes, and the shape thereof is not limited to a specific shape.

When the plurality of collection recesses 323 or the plurality of concave and convex parts 321 and 323 is formed at the screw blade 320, as described above, the surface area of the outer edge of the screw blade 320 is increased, which assists in increasing the amount of the specimen that is collected.

In the concave and convex parts 321 and 323 of the screw blade 320, the specimen may be held in the collection recesses 323, which are concave portions 323. Here, the specimen may be held due to viscosity of the specimen, elasticity of the flexible screw blade 320, and an increase in surface area of the screw blade 320 that contacts the specimen.

FIG. 3 illustratively shows an example in which an angularly concave and convex structure is formed at the outer edge of the screw blade; however, the present invention is not limited thereto. For example, curved concave parts and curved convex parts may be alternately formed at the outer edge of the screw blade 320 in the shape of a sine wave, which is also preferable.

For reference, reference symbol AX indicates the central axis of the support unit 200 or the hub 310 in the longitudinal direction thereof.

It is preferable for the thickness of the screw blade 320 from one end of the screw blade 320 connected to the outer circumferential surface of the hub 310 to the other end of the screw blade 320, which is the outer edge of the screw blade 320, to be uniform. In addition, as shown in FIG. 4, it is also preferable for the thickness of a part of the screw blade to be reduced.

FIG. 4 is a partial side sectional conceptual view schematically showing the hub and the screw blade of the specimen collection unit of the specimen collection stick according to the embodiment of the present invention. That is, this figure is a conceptual view showing a plane including a point on the outer edge of the screw blade 320 and the central axis of the hub 310 in the longitudinal direction thereof as a side sectional view.

In FIG. 4, one side of the screw blade 320 is connected to the hub 310. Here, the outer edge of the screw blade 320 is the other end of the screw blade 320. The screw blade 320 may also be formed such that at least a part LD2 of a longitudinal length section L0 has a thickness (T0 -> TE) gradually reduced to the other side of the screw blade.

Here, reference symbol T0 indicates the thickness T0 at the point of the at least a part LD2 of the longitudinal length section L0 of the screw blade 320 from which the thickness starts to be gradually reduced to the other side of the screw blade, and reference symbol TE indicates the thickness TE at the other end of the screw blade 320.

That is, it is preferable for the at least a part LD2 of the longitudinal length section L0 to be formed such that the thickness is reduced from T0 to TE. In other words, it is preferable for the thickness to be tapered.

As described above, it is preferable for the screw blade 320 to be formed such that the lateral width WD or the thickness (T0 -> TE) of the at least a part LD2 of the longitudinal length section L0 is gradually reduced to the other side of the screw blade.

It is preferable for the thickness of the screw blade 320 to range from 0.1 mm to 1 mm, more preferably from 0.2 mm to 0.8 mm.

The screw blade 320 may be made of a polymer material that exhibits flexibility and elasticity or an elastomer.

When the screw blade 320 is formed such that the thickness is gradually reduced to the outer edge of the screw blade, i.e. the other end of the screw blade, as described above, the outer edge of the screw blade 320 is capable of holding a specimen brought into contact therewith while returning to the original state thereof after being flexibly bent, which is preferable.

Since the specimen brought into contact with the spiral screw blade 320 disposed around the hub 310 or adhered thereto remains held between the pitches of the screw blade 320 while the specimen is inhibited from being separated from the screw blade 320, as described above, it is possible to further increase the amount of the specimen that is collected, compared to the conventional art. Furthermore, since the concave portions 323, which are the collection recesses 323, and the convex portions 321 are alternately formed at the outside of the screw blade 320, the specimen brought into contact therewith or adhered thereto may remain held in the collection recesses 323 while the specimen is inhibited from being separated therefrom, whereby it is possible to increase the amount of the specimen that is collected.

It is preferable for the screw blade 320 to be manufactured by molding such that screw blade has a size set depending on the part of the body of the subject into which the screw blade is inserted, such as the nasal cavity, the oral cavity, the anus, or the cervix. The specimen collection unit 300 including the screw blade 320 and the hub 310 may be made of a polymer material that exhibits flexibility and elasticity or an elastomer.

Meanwhile, the following embodiment may also be provided.

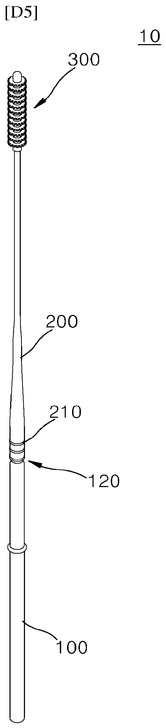

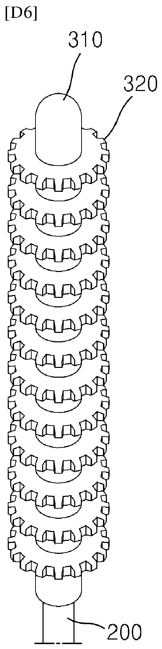

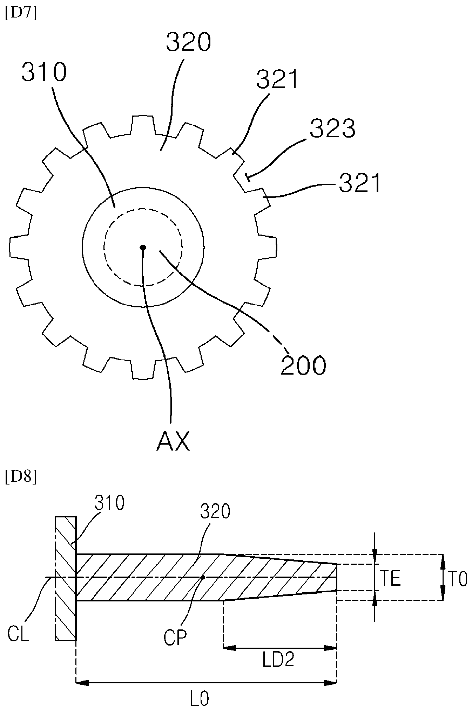

FIG. 5 is a perspective view schematically showing a specimen collection stick according to an applied embodiment of the present invention, FIG. 6 is a perspective view schematically showing a specimen collection unit of the specimen collection stick according to the applied embodiment of the present invention, and FIG. 7 is a front view schematically showing the specimen collection unit of the specimen collection stick according to the applied embodiment of the present invention.

Referring to FIGs. 5 to 7, the specimen collection stick according to the embodiment of the present invention includes a support unit 200 and a specimen collection unit 300, and may further include a handle unit.

First, the support unit 200 is formed so as to have a shape, such as a rod or a bar, and has a predetermined length, as shown in the figures. The specimen collection unit 300 is coupled to a front end of the support unit 200. The support unit 200 supports the specimen collection unit 300. The handle unit is connected to the rear of the support unit 200. The support unit 200 supports the specimen collection unit 300 such that the specimen collection unit can be introduced into the body of a subject.

It is sufficient for the support unit 200 to support the specimen collection unit 300 such that the specimen collection unit can be introduced into the body of the subject in order to perform specimen collection, and the shape of the support unit is not limited to a specific shape.

Furthermore, it is preferable for the support unit 200 to be made of a polymer material that exhibits flexibility and elasticity such that the support unit can return to the original shape thereof while being flexibly deformed by force applied thereto.

Meanwhile, when the specimen collection unit 300 and the support unit 200 of the specimen collection stick 10 are withdrawn from a specimen container (not shown) using a tool, such as a pincette, it is necessary to wash the pincette in advance. In addition, after the support unit 200 of the specimen collection stick 10 is withdrawn from the specimen container using the pincette and the specimen collection unit 300 and the support unit 200 of the specimen collection stick 10 are located at a position intended by an examiner, it is necessary to wash the used pincette again.

Since it is necessary to wash the pincette whenever the pincette is used in the process of withdrawing the specimen collection stick from the specimen container, as described above, specimen examination efficiency is lowered. In order to withdraw the specimen collection unit 300 and the support unit 200 of the specimen collection stick 10 from the specimen container without using the pincette, therefore, the following construction may be provided.

That is, a coupling projection or coupling protrusion 210 may be formed at an outer surface of the support unit 200 so as to protrude from the outer surface thereof such that the coupling projection or coupling protrusion is fixed to a cover or lid of the specimen container by coupling.

The cover or lid (not shown) of the specimen container (not shown) may be provided at an inside thereof with a fixing groove or fitting projection (not shown) configured to allow the support unit 200 of the specimen collection stick to be fixed to the cover or lid (not shown) of the specimen container therethrough. The coupling projection or coupling protrusion 210 of the support unit may be coupled to the fixing groove or fitting projection provided at the inside of the cover or lid of the specimen container by engagement or fitting.

Since the support unit 200 can be fixed to the cover or lid of the specimen container, as described above, it is possible to easily withdraw the support unit 200 from the specimen container together with the cover or lid of the specimen container when the cover or lid is removed from the specimen container without using a tool, such as a pincette, which is preferable.

FIG. 5 shows a ring-shaped coupling projection or coupling protrusion 210 formed on the outer surface of the support unit 200 in a circumferential direction thereof as an illustrative example of the coupling projection or coupling protrusion 210. As shown in FIG. 5, it is also preferable for one or more ring-shaped coupling projections or coupling protrusions 210 to be provided on the outer surface of the support unit 200 at the rear of the support unit 200.

As long as the support unit 200 can be coupled to the cover of the specimen container, as described above, it is possible to improve convenience in use, which is preferable.

As previously described, the handle unit 100 is located at the rear of the support unit 200. That is, a rear end of the support unit 200 is connected to the handle unit 100.

The handle unit 100 is connected to the rear end of the support unit 200, and the handle unit has a predetermined length such that the handle unit can be gripped by the hand or fingers of the examiner.

As shown in the figures, the handle unit 100 is formed in the shape of a rod or a bar having a predetermined length. The handle unit 100 is gripped by the hand or fingers of the examiner. The examiner may perform control such that the specimen collection unit 300 is introduced or inserted into the body of the subject using the handle unit 100.

Consequently, the specimen collection unit 300 may be introduced or inserted into the body of the subject through the oral cavity or the nasal cavity of the subject under control of the examiner using the handle unit 100.

As shown in the figures, it is preferable for a segment groove 120, such as a segment joint, capable of distinguishing between the handle unit 100 and the support unit 200 to be provided between the handle unit 100 and the rear end of the support unit 200. That is, it is preferable for the segment groove 120 to be provided between the handle unit 100 and the support unit 200 such that the handle unit 100 and the support unit 200 can be separated from each other.

After the specimen collection unit 300 is withdrawn from the body of the subject according to the intention of the examiner, who is a user, the specimen collection unit 300 and the support unit 200 are received in the specimen container.

Since it is not necessary to receive the handle unit 100 in the specimen container at this time, it is preferable to separate the handle unit 100 from the support unit 200. As shown in the figures, therefore, it is preferable for the segment joint or segment groove 120 to be provided between the support unit 200 and the handle unit 100 such that the support unit 200 and the handle unit 100 can be separated from each other as needed.

After the support unit 200 and the handle unit 100 are separated from each other, the support unit 200 may be coupled to the cover or lid of the specimen container, as previously described. It is preferable for the coupling projection or coupling protrusion 210 to be formed at an outer circumferential surface of the support unit so as to protrude therefrom or for a lid fastening groove to be formed in the outer circumferential surface of the support unit such that the support unit can be fixed to the cover or lid of the specimen container, in which the specimen collection unit is received, by coupling.

The specimen collection unit 300 is a part that is inserted or introduced into the body of the subject so as to come into contact with a specimen. That is, the specimen collection unit is a part that collects the specimen. The specimen collection unit 300 is located at the front end of the support unit 200 in order to gather or collect a specimen in the body of the subject that comes into contact with the specimen collection unit.

The specimen collection unit 300 includes a hub 310 and a screw blade 320.

The hub 310 is coupled to the front of the support unit 200. It is preferable for the hub 310 and the support unit 200 to be coupled to each other such that a central axis of the hub in a longitudinal direction thereof and a central axis of the support unit 200 in a longitudinal direction thereof are aligned with each other. The hub 310 is coupled to or formed at the support unit 200 such that a front portion of the support unit 200 is inserted into the hub 310. In addition, the hub 310 supports the screw blade 320 disposed at the outside thereof.

The screw blade 320 is disposed at the outside of the hub 310, is supported by the hub 310, and is spirally formed along the central axis of the hub in the longitudinal direction thereof.

The spirally formed screw blade 320 is disposed at the outside of the hub 310 such that a specimen brought into contact with the screw blade is withdrawn from the body of the subject in a state of being adhered to or held by the screw blade.

It is preferable for the hub 310 to be made of a flexible or elastic material. That is, the hub may be made of a polymer material that exhibits flexibility and elasticity or an elastomer material.

As shown in the figures, the hub 310 may be formed in the shape of a bar having a circular section; however, the present invention is not limited thereto.

A plurality of collection disks 320 arranged in the longitudinal direction of the hub is configured to allow a specimen brought into contact therewith to be adhered thereto or to be held thereby and to be withdrawn from the body of the subject in that state. It is preferable for the plurality of collection disks 320 to be spaced apart from each other by a predetermined distance. Also, it is preferable for the center of each of the collection disks 320 to be on the central axis of the hub in the longitudinal direction thereof.

Each of the collection disks 320 is formed in the shape of a plate having at least one curved or flat side surface. In addition, the edge of the collection disk 320 is circular or oval. The collection disk 340 may have a disk or circular shape.

The collection disks 320 may be formed around the hub 310 at the outer circumferential surface of the hub 310. The collection disks 320 thus formed are supported by the hub 310.

The hub 310, which supports the collection disks 320, is coupled to the front portion of the support unit 200. Consequently, the hub 310 supports the collection disks 320 while being supported by the support unit 200. When the collection disks 320 are included in the specimen collection unit 300, as described above, the surface area of the specimen collection unit 300 that comes into contact with a specimen is increased. Consequently, the amount of a specimen that is collected is increased.

A gap, through which the outer circumferential surface of the hub 310 is exposed outwards, is provided between neighboring ones of the collection disks 320. Even in the gap, the specimen collection unit may come into contact with a specimen in the body of the subject, and the specimen may be withdrawn from the body of the subject in a state of being held in the gap.

In short, a specimen may be adhered and held by the outer circumferential surface of the hub between neighboring ones of the collection disks 320, and the specimen may be withdrawn from the body of the subject in that state.

Each of the collection disks 320 is flexible and elastic. Consequently, each of the plurality of collection disks 320 abuts bodily tissue in the body of the subject. The examiner swings the specimen collection unit 300 in the body of the subject using the handle unit 100 such that the collection disks 320 can scrape or sweep the bodily tissue in which the specimen is present while abutting the bodily tissue. As a result, the specimen is adhered to the surface of each of the collection disks 320 or is held in the gap between the neighboring ones of the collection disks 320.

That is, the specimen is adhered to the surface of each of the collection disks 320 or is held in the gap between the neighboring ones of the collection disks 320 in the state in which the specimen collection unit 300 is inserted in the body of the subject, whereby the specimen remains on the specimen collection unit 300 when the specimen collection unit 300 is withdrawn from the body of the subject.

The gap between the neighboring ones of the collection disks 320 may also be referred to as a collection space or a collection gap.

Also, in at least some of the plurality of collection disks 320, it is also preferable for neighboring ones of the collection disks 320 to have different diameters or different long radii. Of course, all of the plurality of collection disks 320 may also have the same diameter or the same long radius.

In the specimen collection unit 300, therefore, the specimen brought into contact with the collection disks 320 or the hub 310 may be maintained in a state of being held by or adhered to the collection disks 320.

As shown in FIGs. 6 and 7, it is preferable for a plurality of concave parts 323 corresponding to the collection recesses and a plurality of convex parts 321 to be alternately formed at the outer edge of each of the collection disks 320. In other words, a plurality of collection recesses 323 may be formed at the outer edge of each of the collection disks 320, which is preferable. Here, the concave and convex parts 321 and 323 or the collection recesses 323 may have various shapes, and the shape thereof is not limited to a specific shape.

When the plurality of collection recesses 323 or the plurality of the concave and convex parts 321 and 323 is formed at each of the collection disks 320, as described above, the surface area of the outer edge of the collection disk 320 is increased, which assists in increasing the amount of the specimen that is collected.

In the concave and convex parts 321 and 323 of the collection disk 320, the specimen may be held in the collection recesses 323, which are concave portions 323. Here, the specimen may be held due to viscosity of the specimen, elasticity of the flexible collection disk 320, and an increase in surface area of the collection disk 320 that contacts the specimen.

FIG. 7 illustratively shows an example in which an angularly concave and convex structure is formed at the outer edge of the collection disk; however, the present invention is not limited thereto. For example, curved concave parts and curved convex parts may be alternately formed at the outer edge of the screw blade 320 in the shape of a sine wave, which is also preferable.

For reference, reference symbol AX indicates the central axis of the support unit 200 or the hub 310 in the longitudinal direction thereof.

It is preferable for the thickness of the collection disk 320 from one end of the collection disk 320 connected to the outer circumferential surface of the hub 310 to the other end of the collection disk 320, which is the outer edge of the collection disk 320, to be uniform. In addition, as shown in FIG. 8, it is also preferable for the thickness of a part of the collection disk to be reduced.

FIG. 8 is a partial side sectional conceptual view schematically showing the hub and the collection disk of the specimen collection unit of the specimen collection stick according to the embodiment of the present invention. That is, this figure is a conceptual view showing a plane including a point on the outer edge of the collection disk 320 and the central axis of the hub 310 in the longitudinal direction thereof as a side sectional view.

In FIG. 8, one side of the collection disk 320 is connected to the hub 310. Here, the outer edge of the collection disk 320 is the other end of the collection disk 320. The collection disk 320 may also be formed such that at least a part LD2 of a longitudinal length section L0 has a thickness (T0 -> TE) gradually reduced to the other side of the collection disk.

Here, reference symbol T0 indicates the thickness T0 at the point of the at least a part LD2 of the longitudinal length section L0 of the collection disk 320 from which the thickness starts to be gradually reduced to the other side of the collection disk, and reference symbol TE indicates the thickness TE at the other end of the collection disk 320.

That is, it is preferable for the at least a part LD2 of the longitudinal length section L0 to be formed such that the thickness is reduced from T0 to TE. In other words, it is preferable for the thickness to be tapered.

As described above, it is preferable for the collection disk 320 to be formed such that the lateral width WD or the thickness (T0 -> TE) of the at least a part LD2 of the longitudinal length section L0 is gradually reduced to the other side of the collection disk.

It is preferable for the thickness of the collection disk 320 to range from 0.1 mm to 1 mm, more preferably from 0.2 mm to 0.8 mm.

The collection disk 320 may be made of a polymer material that exhibits flexibility and elasticity or an elastomer.

When the collection disk 320 is formed such that the thickness is gradually reduced to the outer edge of the collection disk, i.e. the other end of the collection disk, as described above, the outer edge of the collection disk 320 is capable of holding a specimen brought into contact therewith while returning to the original state thereof after being flexibly bent, which is preferable.

Since the specimen brought into contact with the plurality of collection disks 320 arranged in the longitudinal direction of the hub 310 or adhered thereto remains held between the collection disks 320 while the specimen is inhibited from being separated from the screw blade 320, as described above, it is possible to further increase the amount of the specimen that is collected, compared to the conventional art. Furthermore, since the concave portions 323, which are the collection recesses 323, and the convex portions 321 are alternately formed at the outside of the collection disk 320, the specimen brought into contact therewith or adhered thereto may remain held in the collection recesses 323 while the specimen is inhibited from being separated therefrom, whereby it is possible to increase the amount of the specimen that is collected.

It is preferable for the collection disk 320 to be manufactured by molding such that the screw blade has a size set depending on the part of the body of the subject into which the screw blade is inserted, such as the nasal cavity, the oral cavity, the anus, or the cervix. The specimen collection unit 300 including the collection disks 320 and the hub 310 may be made of a polymer material that exhibits flexibility and elasticity or an elastomer.

In the specimen collection stick according to the embodiment of the present invention, as described above, the amount of the specimen that is collected by the screw blade or the collection disks included in the specimen collection unit 300 is greater than the amount of the specimen collected by the conventional swab for specimen collection.

Consequently, it is possible to increase the amount of the specimen that is collected from the subject and to increase the amount of the collected specimen that is dissolved or dispersed in a reagent or a solution from the specimen collection stick.

As can be seen from the above description, the specimen collection stick according to the present invention has advantages in that a flocking process, which is required to manufacture the conventional swab for specimen collection, is not necessary, whereby it is possible to reduce the manufacturing cost of the specimen collection stick, and in that there is no fiber layer in the specimen collection stick, whereby it is possible to prevent a problem in which the fiber layer is separated from the specimen collection stick and remains in the body of the subject, unlike the conventional swab for specimen collection.

In addition, the specimen collection stick according to the present invention has advantages in that it is possible to increase the amount of the specimen that is collected from the subject, compared to the conventional swab, to increase the amount of the collected specimen that is dissolved or dispersed in a reagent or a solution from the specimen collection stick, and to improve examination accuracy with an increase in amount of the specimen that is collected.

Furthermore, the specimen collection stick according to the present invention has advantages in that it is possible to shorten time necessary for specimen collection and to reduce inconvenience of the subject during specimen collection. In addition, the specimen collection unit, which is inserted into the oral cavity or the nasal cavity of the subject, is made of a polymer material that exhibits flexibility and elasticity or an elastomer material, whereby it is possible to reduce inconvenience of the user.

Although the present invention has been described in detail based on the embodiment with reference to the accompanying drawings, as described above, the embodiments are merely provided to describe the present invention, and therefore it should be understood that the present invention is not limited to the embodiments, and the scope of rights of the present invention should be interpreted by the following claims and equivalent thereto.

- 100:: Handle unit
- 200:: Support unit
- 300:: Specimen collection unit
- 310:: Hub
- 320:: Collection disk, screw blade

## Claims

1. A specimen collection stick comprising:
a rod-shaped or bar-shaped support unit having a predetermined length; and
a specimen collection unit located at a front end of the support unit, the specimen collection unit being configured to collect a specimen in a body of a subject that comes into contact with the specimen collection unit, wherein
the specimen collection unit comprises:
a hub coupled to a front of the support unit; and
a screw blade disposed on an outer surface of the hub, the screw blade being supported by the hub, the screw blade being spirally formed along a central axis of the hub in a longitudinal direction thereof,
a plurality of concave parts corresponding to collection recesses and a plurality of convex parts are alternately formed at an outer edge of the screw blade, and
the screw blade or the hub is made of a flexible or elastic material.

2. The specimen collection stick according to claim 1, wherein a coupling projection or coupling protrusion is formed at an outer circumferential surface of the support unit so as to protrude therefrom, or a lid fastening groove is formed in the outer circumferential surface of the support unit, such that the support unit can be fixed to a lid of a specimen container configured to receive the specimen collection unit therein by coupling.

3. A specimen collection stick comprising:
a rod-shaped or bar-shaped support unit having a predetermined length; and
a specimen collection unit located at a front end of the support unit, the specimen collection unit being configured to collect a specimen in a body of a subject that comes into contact with the specimen collection unit, wherein
the specimen collection unit comprises:
a hub coupled to a front of the support unit; and
a plurality of collection disks disposed on an outer surface of the hub, the collection disks being supported by the hub, the collection disks being arranged in a longitudinal direction of the hub,
a plurality of concave parts corresponding to collection recesses and a plurality of convex parts are alternately formed at an outer edge of each of the plurality of collection disks, and
each of the collection disks or the hub is made of a flexible or elastic material.

4. The specimen collection stick according to claim 3, wherein a coupling projection or coupling protrusion is formed at an outer circumferential surface of the support unit so as to protrude therefrom, or a lid fastening groove is formed in the outer circumferential surface of the support unit, such that the support unit can be fixed to a lid of a specimen container configured to receive the specimen collection unit therein by coupling.
